# EUROPEAN PATENT APPLICATION

(11) **EP 4 538 297 A1**
(43) Date of publication of application: **16.04.2025**
(21) Application number: 24829357.3
(22) Date of filing: 28.06.2024
(51) Int. Cl.: C07K 19/00, C12N 15/85, A61K 39/39, A61K 39/00

(54) **GLYCOSYL-MODIFIED FUSION PROTEIN, NUCLEIC ACID MOLECULE, EXPRESSION VECTOR, HOST CELL AND USE**

(30) Priority: 30.06.2023 CN 202310798756; 30.06.2023 CN 202310807755
(71) Applicant: Kaimi Biomedicine (Chengdu) Co., Ltd., Chengdu Tianfu International Bio-town Chengdu Sichuan 610219 (CN)
(72) Inventor: SHI, Yuenian, NY 11577 (US); YU, Bo, Kendall Park, NJ 08824 (US); LU, Hanxin, Chengdu, Sichuan 610219 (CN)
(74) Representative: Ipside
(86) International application number: PCT/CN2024/102295
(87) International publication number: WO 2025/002321

(57) **Abstract**

This application provides a glycosyl-modified fusion protein, a nucleic acid molecule, an expression vector, a host cell and use thereof. A first aspect of this application provides a glycosyl-modified fusion protein including a murine Fc variant and a prostate cancer antigen, where the murine Fc variant is obtained by subjecting a murine Fc fragment to amino acid mutation and non-mammalian glycosylation-modification; the murine Fc variant includes at least one of alanine at position 223, alanine at position 228, alanine at position 230, leucine at position 330 and glutamic acid at position 332; the non-mammalian glycosylation-modification excludes sialic acid-modification; the position numbering is performed according to the EU numbering system; the murine Fc variant enhances the binding of the murine Fc fragment to DC cells and DC activation, including the proliferation and activation of specific T cells.

## Description

This application claims priority to Chinese patent application No. 202310798756.4, entitled "FC VARIANT, FUSION PROTEIN AND APPLICATION THEREOF" and filed with the China National Intellectual Property Administration on June 30, 2023; and Chinese patent application No. 202310807755.1, entitled "CHIMERIC FUSION PROTEIN, NUCLEIC ACID MOLECULE, EXPRESSION VECTOR, HOST CELL AND APPLICATION" and filed with the China National Intellectual Property Administration on June 30, 2023. Both of the aforementioned applications are hereby incorporated by reference in their entireties.

### TECHNICAL FIELD

This application relates to a glycosyl-modified fusion protein, a nucleic acid molecule, an expression vector, a host cell and application thereof, and relates to a field of biopharmaceutical technology.

### BACKGROUND

Adaptive immune response is generally divided into two major categories: humoral immunity, which is primarily mediated by circulating antibodies, and cellular immunity, which is mediated by various forms of cells. When the host immune system develops tolerance to tumor antigens, the tumor antigens are recognized as "self-antigens" and are not recognized by antigen-presenting cells (APC), making it impossible to activate the immune system to fight tumor diseases. Therefore, introducing "new antigens" into the immune system is a method of treating cancer.

Prostate cancer (PCa) is one of the most common malignancies in males in the world. Currently, androgen deprivation therapy (ADT) is still the main treatment method for advanced cases. However, these patients often acquire metastasis or castration resistance, causing the tumor to proceed to castration-resistant prostate cancer (CRPC), which is more malignant and has a worse prognosis. Currently, a variety of androgen receptor antagonists (ARs) have been authorized by the FDA for the treatment of CRPC, but still showing a limited curative effect. In addition, new drugs such as antibody-drug conjugates (ADCs) targeting prostate adenocarcinoma markers such as PSMA and PAP, and chimeric antigen receptor T (CAR-T) cells are also constantly developing as alternative means of treating prostate cancer. Traditional or new therapy for patients with prostate adenocarcinoma is still extremely challenging. "Recombinanting" or "reactivating" the body's immune response may provide a new opportunity for curing patients with prostate cancer.

Dendritic cells (DCs) are the most powerful APCs and become an important link in the initiation of tumor antigen-specific T lymphocyte immune response and humoral immune response. After the tumor antigen forms a complex with the antibody, the Fc fragment of the antibody molecule has a higher affinity with the Fc receptor, making it easier for the tumor antigen bound to the antibody to bind to APC, activating B lymphocytes and T lymphocytes, and resulting in humoral and cellular immune responses. For example, ovarian cancer Mab-B43.13, PSA antibody or HBV antibody-antigen complex is more easily recognized by APC or DC, and activate immune killing after antigen presentation.

Human FcγRIIa (hFcγRIIA) is an activating Fc receptor. When FcγRIIa binds to Fc fragment of an antibody, activation signals can be transmitted, mediating the functions of Fc fragment effector, including cytolysis, phagocytosis, degranulation, and production of cytokine. Meanwhile, FcγRIIa can regulate dendritic cell maturation and antigen presentation, mediate T cell memory effects, participate in stimulating protective CD8+T cell responses, and promote long-term protection against viral infections, thereby achieving the effect of both clearing target cells and maintaining long-term immune protection.

In order to gain insight into the binding between the Fc fragment and FcγRIIa, many researchers have modified the structure of the Fc fragment. Some researchers have found that the Fc fragment mutation (G236A) selectively activates FcγRIIa on the basis of inhibiting FcγRIIb (the two have 96% sequence identity), thereby enhancing ADCP mediated by monocyte-derived macrophages. For S239D/I332E mutation of the Fc fragment, addition of G236A can increase the binding force of FcγRIIa by 70 times, increase the binding ratio of FcγRIIa/FcγRIIb (activation/inhibition ratio) by 13 times, and enhance the phagocytosis of antibody-coated target cells by macrophages (Richards J O, K.S., Lazar G A, et al., Molecular cancer therapeutics, 2008, 7(8): 2517-2527, Optimization of antibody binding to FcγRIIa enhances macrophage phagocytosis of tumor cells[J]. Molecular cancer therapeutics, 2008. 7(8): p. 2517-2527). Other researchers have found that Fc mutations induce the activation of T cell responses of CD8+ and CD4+ T cells (Bournazos, S. et al., Fc-optimized antibodies elicit CD8 immunity to viral respiratory infection, Nature, Vol. 588: 17, December 2020). Therefore, the modification to the Fc fragment may affect the effector function of the Fc fragment, thereby changing the biological activity of the antibody.

The glycosylation pathway of insects is different from that of higher eukaryotes. The recombinant N-glycoproteins commonly expressed have simple sugar chain which has no sialylated high/low mannose types, rather than the sialylated complex sugar chains produced at the same glycosylation position by the mammals. This may be due to its cells having high N-acetylglucosaminidase activity, low glycosyltransferase activity and fewer sugar-nucleoside sources. Effector functions elicited by the fragment crystallizable (Fc) region of immunoglobulin G (IgG) antibodies are altered by the presence of a terminal sialic acid (Sia) residue at position of asparagine-297 (Asn-297). Studies have found that hypersialylation hinders binding to FcγRIIIa of natural killer (NK) cells, resulting in reduced ADCC efficacy. The sugar chains having terminal sialic acid residues and expressed by SF9 cells can also bind to the mannose receptor (CD206), which may increase the ability of dendritic cells for taking up and processing glycoproteins, thereby enhancing the immune response (Scallon B J, Tam S H, McCarthy S G, et al. Higher levels of sialylated Fc glycans in immunoglobulin G molecules can adversely impact functionality[J]. Molecular immunology, 2007, 44(7): 1524-1534).

In addition to a large number of human immunoglobulins, there have also been successful examples of non-humanized antibodies combined with antigens as therapeutic vaccines, such as CA-125+ mouse IgG, Oregovomab (M. Brewer, R. Angioli, G. Scambia, et al., Front-line chemo-immunotherapy with carboplatin-paclitaxel using oregovomab indirect immunization..., Gynecologic Oncology, https://doi.org/10.1016/j.ygyno.2019.12.024). This example shows that the mouse IgG can increase the immune response to the antigen carried. Some researchers have directly linked the Fc fragment of murine IgG1 to various antigens, such as HBV-mFc, producing good effects (Allan Ma et al., A dendritic cell receptor-targeted chimeric immunotherapeutic protein (C-HBV) for the treatment of chronic hepatitis B, HUMAN VACCINES & IMMUNOTHERAPEUTICS 2020, VOL. 16, NO. 4, 756-778).

There are many reports on the modification of the Fc fragment of human immunoglobulins in the literatures, but there are relatively few reports on the modification of the Fc fragment of murine immunoglobulin. In order to determine whether engineering modification can increase the presentation effect, we believe that it is necessary to study the engineering modification of the Fc fragment of murine immunoglobulin.

### SUMMARY

This application provides a glycosyl-modified fusion protein, including a murine Fc variant and a prostate cancer tumor antigen, in which the murine Fc variant is obtained by modifying a murine IgG1 Fc fragment. The fusion protein helps to improve binding of the murine Fc fragment to a dendritic cell (DC) and DC activation, including proliferation and activation of specific T cells.

This application further provides a nucleic acid molecule encoding the above fusion protein, as well as an expression vector and a host cell including the nucleic acid molecule.

This application further provides use of the above fusion protein in treating prostate cancer.

A first aspect of this application provides a glycosyl-modified fusion protein including a murine Fc variant and a prostate cancer tumor antigen, in which the fusion protein improves binding to DC and DC activation, including the proliferation and activation of specific T cells.

The murine Fc variant is obtained by subjecting a murine Fc fragment to amino acid mutation and non-mammalian glycosylation-modification.

The murine Fc variant includes at least one of alanine (Ala) at position 223, alanine (Ala) at position 228, alanine (Ala) at position 230, leucine (Leu) at position 330 and glutamic acid (Glu) at position 332.

The non-mammalian glycosylation-modification excludes sialic acid-modification.

The position numbering of amino acid is based on EU numbering system.

In the solution provided in this application, the murine Fc variant is obtained by amino acid mutation and non-mammalian glycosylation-modification on the basis of a wild-type murine Fc fragment shown in SEQ ID NO: 1.

In addition, the wild-type murine Fc fragment shown in SEQ ID NO: 1 is a murine IgG1 Fc fragment. In addition to the IgG1 Fc fragment, other murine IgG Fc fragments are applicable to this application. For example, when based on a murine IgG2 Fc fragment, an Fc variant obtained after amino acid mutation and non-mammalian glycosylation-modification has a similar effect of improving the binding to DC and DC activation.

In a specific embodiment, the amino acid sequence of the murine Fc variant is shown in SEQ ID NO: 2 or SEQ ID NO: 3.

The amino acid sequence shown in SEQ ID NO: 2 includes 232 amino acid residues. The mutation sites are numbered according to the EU numbering system. The alanine at position 223, alanine at position 228 and at position 230 are respectively located at the positions underlined in the following sequence:

VDKKIVPADCGCAPCICAVPEVSSVFIFPPKPKDVLTITLTPKVTCVVVDISK
DDPEVQFSWFVDDVEVHTAQTQPREEQFNSTFRSVSSELPIMHQDWLNGKEFKCRVN
SAAFPAPIEKTISKTKGRPKAPQVYTIPPPKEQMAKDKVSLTCMITDFFPEDITVEWQW
NGQPAENYKNTQPIMDTDGSYFVYSKLNVQKSNWEAGNTFTCSVLHEGLHNHHTEK
SLSHSPGL.

The alanines at respective positions 223 and 228, at position 230, at position 330 and at position 332 in SEQ ID NO: 3 are respectively located at the positions indicated by underline in the following sequence:

VDKKIVPADCGCAPCICAVPEVSSVFIFPPKPKDVLTITLTPKVTCVVVDISK
DDPEVQFSWFVDDVEVHTAQTQPREEQFNSTFRSVSSELPIMHQDWLNGKEFKCRVN
SAAFPLPEEKTISKTKGRPKAPQVYTIPPPKEQMAKDKVSLTCMITDFFPEDITVEWQ

WNGQPAENYKNTQPIMDTDGSYFVYSKLNVQKSNWEAGNTFTCSVLHEGLHNHHT
EKSLSHSPGL.

Non-mammalian glycosylation is distinguished from mammalian glycosylation by the presence or absence of sialic acid-modification and the content of mannose. In the technical solution provided in this application, the glycosylation is mainly N-glycosylation, and the glycosyl is derived from at least one of mannose, N-acetylglucosamine and fucose. A carbohydrate chain structure formed by linking of glycosyl is one or more selected from high mannose type, oligomannose type and fucose type. The high mannose type is composed of GlcNAc and mannose, and contains 5 to 9 mannoses. The oligomannose type refers to containing less than 5 mannoses. The fucose type refers to containing fucose. In the solution provided in this application, the above-mentioned glycosylation sites are all located at the amino acid of the Fc variant at position 297. The non-mammalian glycosylation helps to improve the binding of fusion proteins to DC and DC activation.

Further, the non-mammal is an insect. Further, the non-mammal is a Spodoptera frugiperda. Still furthermore, the above fusion protein is obtained by expression in Sf9 insect cells.

In the solution provided in this application, a prostate cancer tumor antigen refers to an antigen component present on prostate cancer cells that is different from normal tissue cells. Anti-prostate cancer immune response can be induced to produce in a body through the antigen. Further, the prostate cancer tumor antigen is prostatic acid phosphatase (PAP), which is a glycoprotein in the prostate exocrine secretions that can hydrolyze phosphate esters. Selecting PAP as an antigen polypeptide can effectively produce an immune response to enhance the human body's immune effect on prostate cancer. Specifically, the amino acid sequence of the prostatic acid phosphatase (PAP) is represented as positions 1-354 of the sequence shown in SEQ ID NO: 4.

In the solution provided in this application, the above-mentioned fusion protein mainly includes a prostate cancer tumor antigen capable of inducing the body to produce an immune response and a murine Fc variant that binds to DC. The murine Fc variant can be connected to a C-terminal of a polypeptide antigen, or the polypeptide antigen and the murine Fc variant can be connected via a linking peptide. In a specific embodiment, the amino acid sequence of the linking peptide is at least one of GGGS and SRGGGS.

In the solution provided in this application, the above-mentioned fusion protein may further include a protein tag to facilitate the expression, detection and purification of a target gene. Further, the protein tag may be a His tag, which is connected to an N-terminal of the polypeptide antigen to improve purification efficiency of the fusion protein.

The fusion protein described above is a glycosylated fusion protein, including a prostate cancer tumor antigen capable of inducing a human immune response and a murine Fc variant. The fusion protein can be obtained by expression in insect cells using a baculovirus expression system.

A second aspect of this application provides a nucleic acid molecule encoding a fusion protein of any one of the foregoing.

A third aspect of this application provides a recombinant expression vector including the above-mentioned nucleic acid molecule.

A fourth aspect of this application provides a host cell, including the above-mentioned recombinant expression vector.

A fifth aspect of this application provides a pharmaceutical composition, including a fusion protein of any one of the foregoing and a pharmaceutically acceptable carrier.

In this application, the pharmaceutically acceptable carrier is non-toxic to cells or individuals at the dosage and concentration used. Generally, the physiologically acceptable carrier is a pH buffer solution. Further, the pharmaceutical composition provided in this application is an injection, and the above-mentioned carrier may further include a diluent such as water, ethanol and polyethylene glycol, and an additive such as sodium chloride, glucose or glycerol. In addition, conventional cosolvents, buffers and the like may further be added.

A sixth aspect of this application provides use of a fusion protein of any one of the foregoing in preparation of a medicament for treating prostate cancer.

A seventh aspect of this application provides use of a fusion protein of any one of the foregoing in preparation of a medicament for treating any tumor expressing the PAP tumor antigen.

In a specific embodiment, the tumor to be treated includes any tumor that expresses the PAP tumor antigen.

The therapeutic effect on cancer shows inhibiting the growth of tumor cells and/or limiting the metastasis and diffusion of tumor cells in a subject.

An eighth aspect of this application provides a method for treating prostate cancer, which includes administering the above-mentioned fusion protein to a subject.

The fusion protein provided in this application is formed by fusion of a murine Fc variant and a prostate cancer tumor antigen. The murine Fc variant helps to improve the binding to DC cells and DC activation, and stimulate the human body to produce an immune response; and the fusion protein formed by fusing the murine Fc variant with the tumor antigen can be used as a vaccine to activate the human body's immune response, thereby playing a role in treating tumors.

### BRIEF DESCRIPTION OF DRAWINGS

FIG. 1a is SDS-PAGE identification result of Seq2-Sf9, where M1 is a protein molecular weight marker and BSA is a protein standard.
FIG. 1b is SDS-PAGE identification result of Seq3-Sf9, where M is a protein molecular weight marker and BSA is a protein standard.
FIG. 2a shows BLI measurement results of Seq2-Sf9 and FcγRIIA protein.
FIG. 2b shows BIL measurement results of Seq3-Sf9 and FcγRIIA protein.
FIG. 3 shows measurement results of the binding force of Seq2-293 and Seq3-293 to DC2.4 at different concentrations.
FIG. 4 shows SDS-PAGE gel identification results of a fusion protein Seq5-293, where M1 represents a protein molecular weight marker, R represents a reduced protein, and NR represents a non-reduced protein.
FIG. 5 shows SDS-PAGE gel identification results of a fusion protein Seq5-Sf9.
FIG. 6 shows SDS-PAGE gel identification results of a fusion protein Seq6-Sf9.
FIG. 7a shows expression detection results of CD54 on the cell surface after DCs are loaded with Seq6-Sf9 and Seq7-293 for 48 hours.
FIG. 7b shows expression detection results of CD83 on the cell surface after DCs are loaded with Seq6-Sf9 and Seq7-293 for 48 hours.
FIG. 8a shows expression detection results of CD54 on the cell surface after DCs are loaded with Seq5-Sf9 for 48 hours.
FIG. 8b shows expression detection results of CD83 on the cell surface after DCs are loaded with Seq5-Sf9 for 48 hours.
FIG. 9a shows expression detection results of CD54 on the cell surface after DCs are loaded with Seq5-Sf9 and Seq5-293.
FIG. 9b shows expression detection results of CD83 on the cell surface after DCs are loaded with Seq5-Sf9 and Seq5-293.
FIG. 10a shows expression detection results of CD54 on the cell surface after DCs are loaded with Seq5-Sf9 and Provenge.
FIG. 10b shows expression detection results of CD83 on the cell surface after DCs are loaded with Seq5-Sf9 and Provenge.
FIG. 11a shows proliferation detection results of CD4+T cells from volunteer 1 after DCs are loaded with Seq5-Sf9 and Provenge.
FIG. 11b shows proliferation detection results of CD4+T cells from volunteer 2 after DCs are loaded with Seq5-Sf9 and Provenge.
FIG. 12 shows ELISPOT detection results of IFNγ secreted by CD8+T cells activated by DCs loaded with Seq5-Sf9.
FIG. 13 is protein expression results of PAP in human prostate cancer cell lines PC3 and LNCap detected by Western Blot.
FIG. 14a shows detection results of killing effect of CTL induced by DCs loaded with Seq5-Sf9 and Provenge on PC3 tumor cells.
FIG. 14b shows detection results of killing effect of CTL induced by DCs loaded with Seq5-Sf9 and Provenge on LNCap tumor cells.
FIG. 15a shows titer detection results of anti-Seq5-Sf9 serum antibody of SD rats immunized by subcutaneous injection of Seq5-Sf9 (with doses of 0/2/10/50 ug), where the samples are collected at time points Day 0 (before a first injection), Day 14 (before a second injection), Day 28 (before a third injection) and Day 35 (one week after the third injection), n=6.
FIG. 15b shows titer detection results of serum antibody of SD rats immunized by subcutaneous injection of Seq5-Sf9 (with doses of 0/2/10/50 ug) on Day 14.
FIG. 15c shows titer detection results of serum antibody of SD rats immunized by subcutaneous injection of Seq5-Sf9 (with doses of 0/2/10/50 ug) on Day 28.
FIG. 15d shows titer detection results of serum antibody of SD rats immunized by subcutaneous injection of Seq5-Sf9 (with doses of 0/2/10/50 ug) on Day 35.
FIG. 16a shows ELISPOT detection results of IFNγ secreted by spleen cells of SD rats on Day 35 after immunization with Seq5-Sf9.
FIG. 16b shows ELISPOT detection spot diagram of IFNγ secreted by spleen cells of SD rats after immunization with Seq5-Sf9 on Day 35.
FIG. 17 shows HE pathological staining images of prostate tissue of SD rats after immunization with Seq5-Sf9 on Day 35 (20× magnification).
FIG. 18a shows comparison of expression detection results of CD54 on the cell surface after DCs are each loaded with Seq6-Sf9 and Seq5-Sf9 for 48 hours.
FIG. 18b shows comparison of expression detection results of CD83 on the cell surface after DCs are each loaded with Seq6-Sf9 and Seq5-Sf9 for 48 hours.

### DESCRIPTION OF EMBODIMENTS

In order to make the purpose, technical solutions and advantages of this application clearer, the technical solutions in the embodiments of this application will be clearly and completely described below in combination with the embodiments of this application. Obviously, the described embodiments are only part of the embodiments of this application, not all of the embodiments. Based on the embodiments in this application, all other embodiments obtained by those skilled in this field without any creative work shall fall within the protection scope of this application.

### Example 1 Affinity detection of Fc variant

### 1.1 Acquisition and affinity detection of Fc variants expressed in Sf9 insect cells

In order to enhance the stability of the mouse IgG Fc fragment, mutations are performed at the sites 223, 228 and 230 of the Fc fragment based on the wild-type murine Fc fragment shown in SEQ ID NO: 1. The amino acid sequence after the mutations is shown in SEQ ID NO: 2. In order to enhance the binding of mouse IgG1 Fc fragment to DC and activation, mutations are performed at the sites 223, 228, 230, 330 and 332 of the Fc fragment based on the wild-type murine Fc fragment. The amino acid sequence after mutations is shown in SEQ ID NO: 3.

The gp64 protein is cloned into a pFastBacHTa (Thermo Fisher Scientific; Cat# 10584-027) vector digested with RsrII (New England Biolabs, R051S) to obtain pFastBacHTa-gp64. The DNA sequence corresponding to the amino acid shown in SEQ ID NO: 2 or SEQ ID NO: 3 is synthesized and then subcloned into the pFastBacHTa-gp64 vector digested with Sal I (New England Biolabs, R3138S) and Hind III (New England Biolabs, R0104S) for insect cell expression using a Bac-to-Bac^{®} baculovirus system. The recombinant plasmid is transformed into DH10Bac chemoreceptive Escherichia coli cells, followed by culturing on a fresh LB agar plate containing 50 µg/ml kanamycin, 7 µg/ml gentamicin, 10 µg/ml tetracycline, 100 µg/ml Bluo-gal, and 40 µg/ml IPTG. After overnight incubation, white colonies are picked and recombinant bacmid DNA is isolated according to a standard project. The positive bacmids are transiently transfected into 2 ml of insect Sf9 cells using a transfection reagent, and the cells are incubated in ESF 921 medium for a period of time. The cells and supernatant are collected. Proteins are purified from the supernatant by Protein A, named Seq2-Sf9 and Seq3-Sf9, respectively.

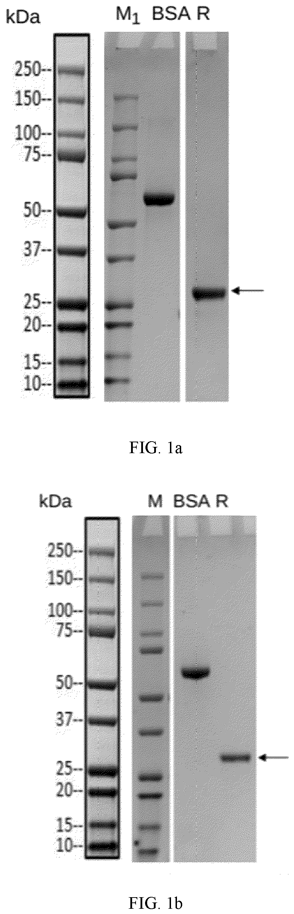

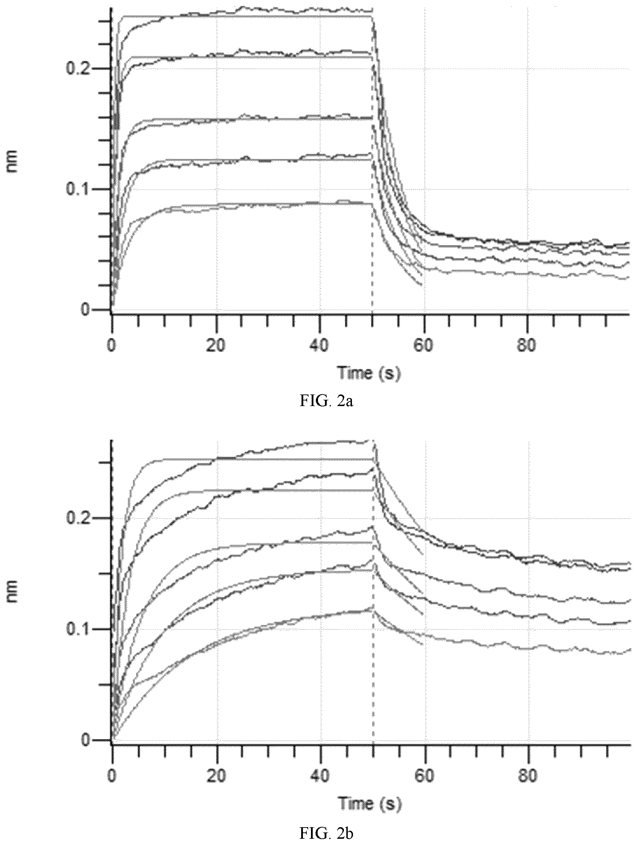

The Seq2-Sf9 and Seq3-Sf9 are identified using Coomassie Brilliant Blue stained SDS-PAGE gel, with BSA as a control protein. The identification results are shown in FIGs. 1a-1b, respectively. The arrows indicate the positions of Seq2-Sf9 and Seq3-Sf9, and it can be determined that the purified protein is obtained with a molecular weight of about 26 kDa. The purified Seq2-Sf9 and Seq3-Sf9 proteins are subjected to multi-concentration affinity detection using biolayer interferometry (BLI), where the concentration gradients are set to 5000nM, 2500nM, 1250nM, 625nM, and 312.5nM to determine the affinity of the Seq2-Sf9 and Seq3-Sf9 proteins to FcγRIIA protein. The detection results are shown in FIGs. 2a-2b, respectively. The curves shown from bottom to top indicate that the concentrations of the Seq2-Sf9 and Seq3-Sf9 proteins decrease in sequence. By calculation, the affinities of the Seq2-Sf9 and Seq3-Sf9 to FcγRIIA protein are 7.893E-07 and 4.496E-07, respectively. The smaller the value, the higher the affinity, indicating that the five mutations in the mouse Fc domain can enhance the affinity to FcγRIIA.

### 1.2 Acquisition of Fc variants expressed by 293 cells and detection of their binding to DC cells

The synthesized DNA sequences corresponding to SEQ ID NO: 2 and SEQ ID NO: 3 are cloned into a eukaryotic expression vector pcDNA3.4 with a secretion signal peptide, with digestion sites of EcoRI and HindIII, and electroporated into Escherichia coli trans5α cells. After screening with ampicillin, the single clone is sequenced to obtain the correct recombinant plasmid. Then, the host bacteria containing the recombinant plasmids are subjected to amplification culture, and sterile endotoxin-free recombinant plasmids are obtained using an endotoxin-removal kit. The sterile endotoxin-free recombinant plasmids are mixed with a polyplus suspension cell transfection reagent, and are transfected into HEK293F cells. After 5 days of amplification culture in a serum-free medium, the culture supernatant is collected, and the proteins Seq2-293 and Seq3-293 are obtained by separation and purification using Protein A resin.

The Seq2-293 and Seq3-293 proteins can enhance the binding to FcR, which is mainly expressed on the surface of various mononuclear cells, including dendritic cells (DC). Since the above Fc variants are from the mouse Fc fragment, the mouse dendritic cell line DC2.4 is selected as the research object, which expresses relatively stable FcR and can bind to the murine Fc. Under normal conditions, Fc has a weak affinity to its receptor FcR. The Seq2-293 and Seq3-293 with a same concentration are incubated with DC2.4 cells at 4°C for 1 h, and biotin-labeled anti-mouse IgG1 Biotin and streptavidin-HRP are added thereinto for reaction respectively, and the reactions are terminated after color development with TMB substrate. Finally, Fc/FcR bound on the surface of DC2.4 cells is indicated by visible light OD450-570 detection.

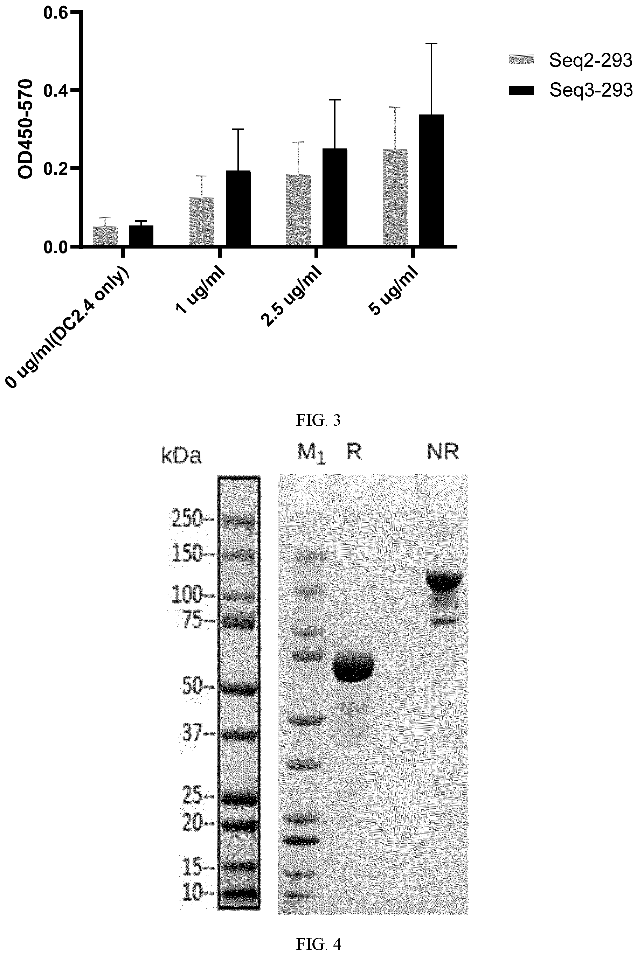

The detection results are shown in FIG. 3. It can be seen that the affinity of Seq3-293 to DC2.4 is significantly higher than that of Seq2-293 at the same concentration, and is proportional to the working concentration. That is, the Fc variant with mutations of Seq3 sequence can enhance the binding to DC and has considerable antigen presentation potential.

### Example 2 Preparation and biological activity evaluation of Seq5-293, Seq5-Sf9, and Seq6-Sf9

### 2.1 Preparation of Seq5-293

The mouse Fc with three mutations having the amino acid sequence shown in SEQ ID NO: 2 is subjected to fusion expression with a specific marker PAP for prostate cancer through a linking peptide, obtaining a fusion protein having the amino acid sequence shown in SEQ ID NO: 4, where positions 1-354 refer to the specific marker PAP for prostate cancer, and positions 361-592 refer to the mouse Fc with three mutations having the amino acid sequence shown in SEQ ID NO: 2.

A gene encoding the fusion protein having the amino acid sequence shown in SEQ ID NO: 4 is synthesized, and the gene sequence encoding the fusion protein is connected to an expression vector pcDNA3.4 vector with digestion sites EcoRI and HindIII to prepare a recombinant expression plasmid. The recombinant expression plasmid is transiently transfected into eukaryotic cells HD293F, and the transfected cells are cultured, and the cell culture is collected, centrifuged and filtered. The filtered culture supernatant is loaded onto a Protein A affinity chromatography column for purification to obtain a fusion protein named Seq5-293. Biochemical arrays are performed using Coomassie Brilliant Blue stained SDS-PAGE gel, and the detection results are shown in FIG. 4.

### 2.2 Preparation of Seq5-Sf9

The mouse Fc with three mutations having the amino acid sequence shown in SEQ ID NO: 2 is subjected to fusion expression with the specific marker PAP for prostate cancer via a linking peptide to obtain a fusion protein having the amino acid sequence shown in SEQ ID NO: 4.

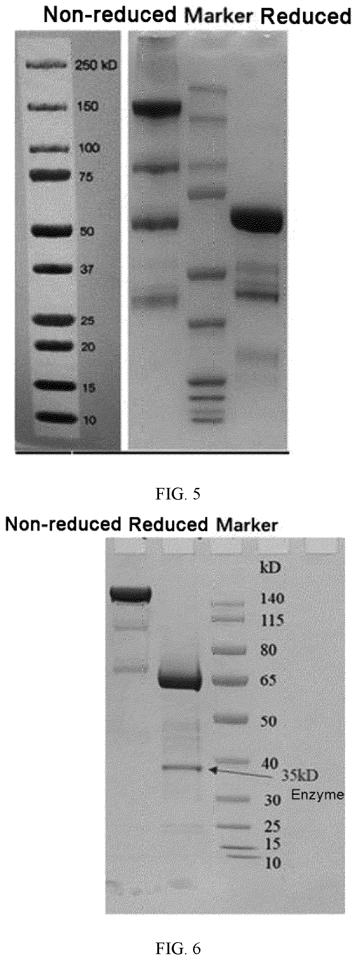

The encoding sequence of the above fusion protein is connected into the pFastBacHTa-gp64 vector digested with Sal I (New England Biolabs, R3138S) and Hind III (New England Biolabs, R0104S) for insect cell expression, which is transformed into Escherichia coli DH10Bac for transposition to produce recombinant baculovirus. The isolated recombinant baculovirus is used to infect the Sf9 insect cells in ESF921 medium. The infected cells are incubated at 27°C for 72 hours. The cells are collected by centrifugation, and lysed by sonication. The fusion protein obtained by purifying the lysate of infected Sf9 cells using a Ni affinity column, is named as Seq4-Sf9. Biochemical arrays are performed using Coomassie Brilliant Blue stained SDS-PAGE gels, and the detection results are shown in FIG. 5.

### 2.3 Preparation of Seq6-Sf9

The mouse Fc with five mutations having the amino acid sequence shown in SEQ ID NO: 3 is subjected to fusion expression with the specific marker PAP for prostate cancer through a linking peptide, obtaining a fusion protein having the amino acid sequence shown in SEQ ID NO: 5, where positions 1-354 refer to the specific marker PAP for prostate cancer, and positions 361-592 refer to the mouse Fc with five mutations having the amino acid sequence shown in SEQ ID NO: 3.

The above fusion protein is expressed in Sf9 insect cells using a same method as the above section 3.2. The expressed fusion protein is named as Seq6-Sf9. Biochemical arrays are performed using Coomassie Brilliant Blue stained SDS-PAGE gel, and the detection results are shown in FIG. 6.

### 2.4 Effect detection of Seq6-Sf9 on DC activation

The human wild-type Fc fragment and the specific marker PAP for prostate cancer are expressed in mammalian 293 cells to obtain Seq7-293 fusion protein, which has an amino acid sequence shown in SEQ ID NO: 6. The effect of the Fc variant and insect glycoform on DC activation is verified using the Seq7-293 fusion protein as a control.

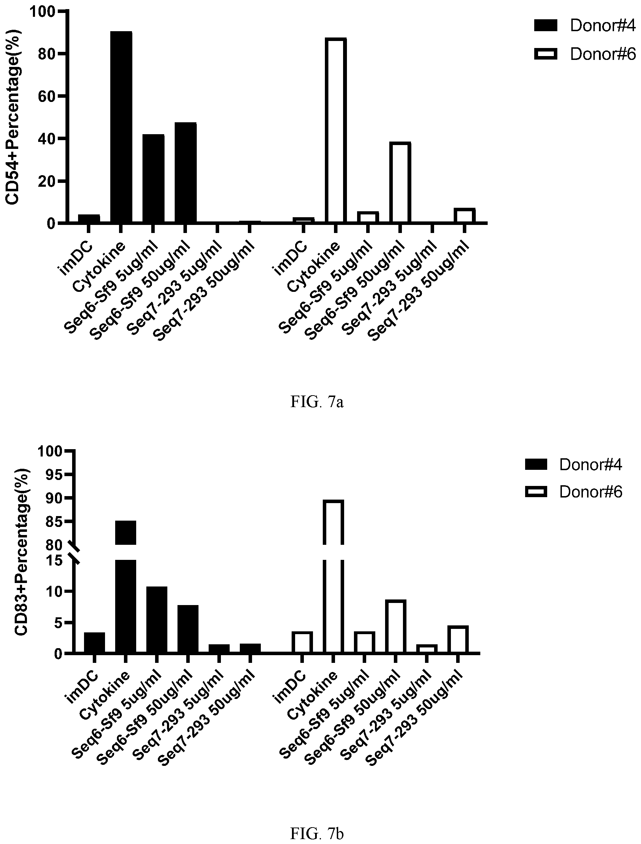

CD14+ monocytes are sorted from PBMCs of two healthy human beings and induced to differentiate into immature DCs in a culture system containing granulocyte-macrophage colony-stimulating factor (GM-CSF) and interleukin-4 (IL-4). The immature DCs have powerful phagocytic function and are loaded with Seq6-Sf9 and Seq7-293 fusion proteins, respectively. DCs phagocytize the fusion proteins and present antigens, thereby activating DCs and expressing activation markers (including CD54 and CD83, etc.). Changes in surface markers of vaccine-loaded DCs are detected by flow cytometry. The detection results are shown in FIGs. 7a-7b. Under the same concentration, compared with Seq7-293, Seq6-Sf9 can significantly activate DCs, and CD54 and CD83 are highly expressed on the cell surface. The above results show that the murine Fc variant and non-mammalian glycoforms in the fusion protein can significantly enhance DC phagocytosis and activation and express activation phenotypes, such as CD54 and CD83.

### 2.5 Effect detection of Seq5-Sf9 on DC activation

### 2.5.1 Evaluation of the immune activation effect of the fusion protein Seq5-Sf9 using DCs induced by CD14+ monocytes in human peripheral blood PBMCs

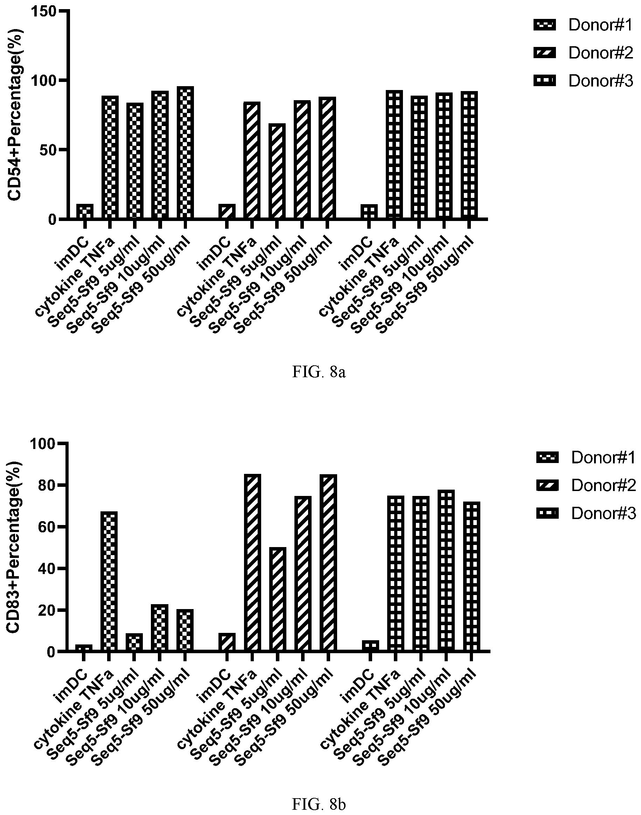

To evaluate the immune activation effect of Seq5-Sf9, CD14+ monocytes are sorted from PBMCs of three healthy human beings and induced to differentiate into immature DCs in a culture system containing granulocyte-macrophage colony-stimulating factor (GM-CSF) and interleukin-4 (IL-4). The immature DCs have powerful phagocytic function, and the uptake function thereof will be enhanced by the murine Fc domain contained in the fusion protein to promote DC maturation and differentiation. Changes in surface markers of vaccine-loaded DCs are detected by flow cytometry, including the adhesion molecule CD54 (ICAM-1: Intercellular adhesion molecule-1), which helps mature DCs to adhere to and interact with other immune cells (such as T cells), and CD83, which is involved in antigen presentation and T cell activation. The detection results are shown in FIGs. 8a-8b. In three different volunteers, CD54 and CD83 are highly expressed on the cell surfaces of DCs loaded with the Seq5-Sf9, indicating that the fusion protein has a potential DC activation effect, activating the antigen presentation ability of DCs loaded with Seq5-Sf9, and initiating an immune response as a starting point.

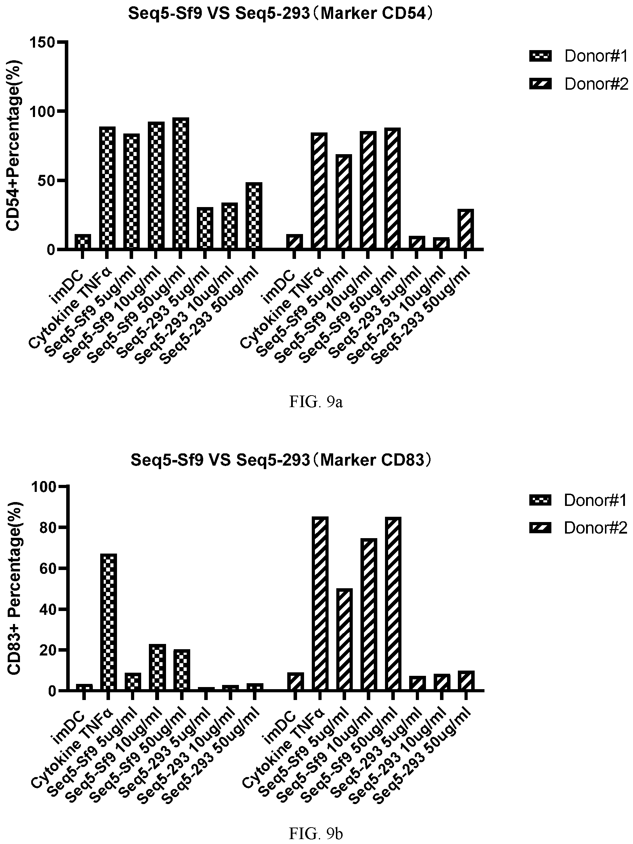

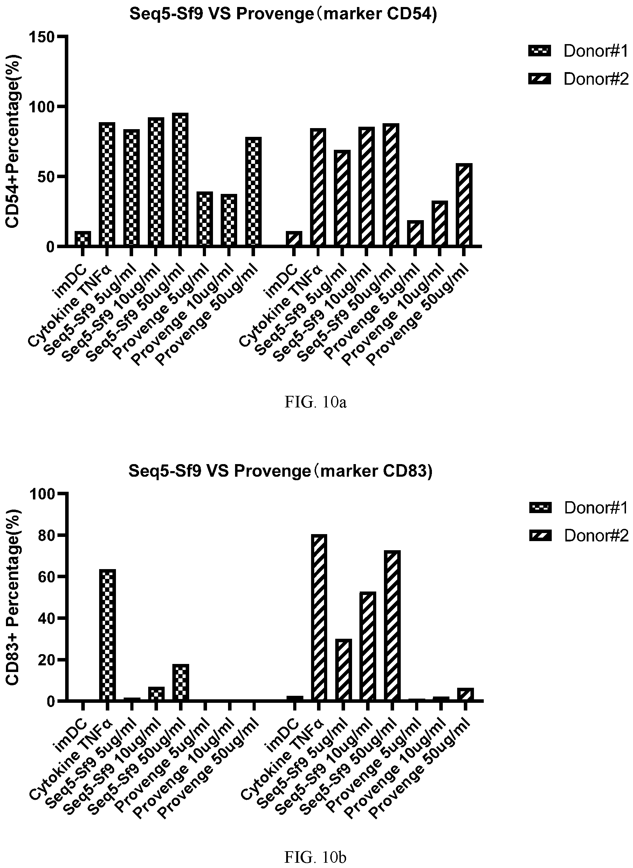

In addition, the immune activation effects of Seq5-Sf9, Seq5-293 and the positive control drug Provenge are compared, where the comparison results are shown in FIGs. 9a-10b. It can be seen that the DC activation effect is enhanced by Fc variants and non-mammalian glycosylation. The Seq5-Sf9 has a stronger DC activation function.

### 2.5.2 In vitro evaluation of key-point for the effect of Seq5-Sf9-loaded DCs on T cell immune response

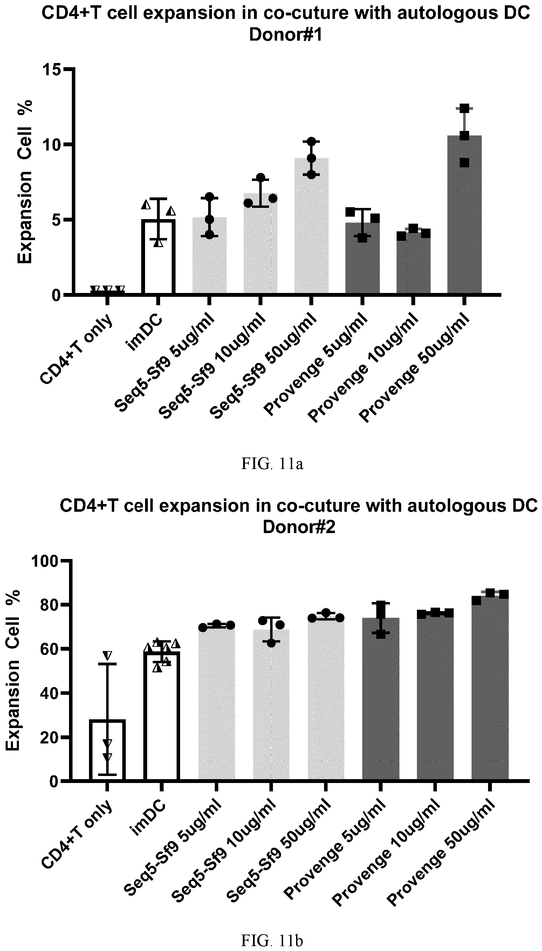

One important function of mature DCs is antigen presentation and activation for CD4+T cell proliferation. The antigen peptide/MHC-I complex or antigen peptide/MHC-II complex on cell surfaces of the mature DCs is recognized by the T cell receptor (TCR). An important step in DC-T activation is T cell proliferation. Therefore, DCs loaded with the Seq5-Sf9 and CD4+T cells labeled by CFSE fluorescent dye are co-cultured. After antigen presentation, T cells could rapidly proliferate and differentiate into Th cells (helper T cells), thereby evaluating the immune conditions based on the proliferation of CD4+T cells. As shown in FIGs. 11a-11b, DCs from different healthy human beings loaded with the Seq5-Sf9 can significantly promote the proliferation of T cells, which is positively correlated with the Seq5-Sf9 concentration. Provenge has the same effect under the same experimental conditions.

### 2.5.3 In vitro evaluation of antigen presentation of Seq5-Sf9-loaded DCs for activated cytotoxic T cells

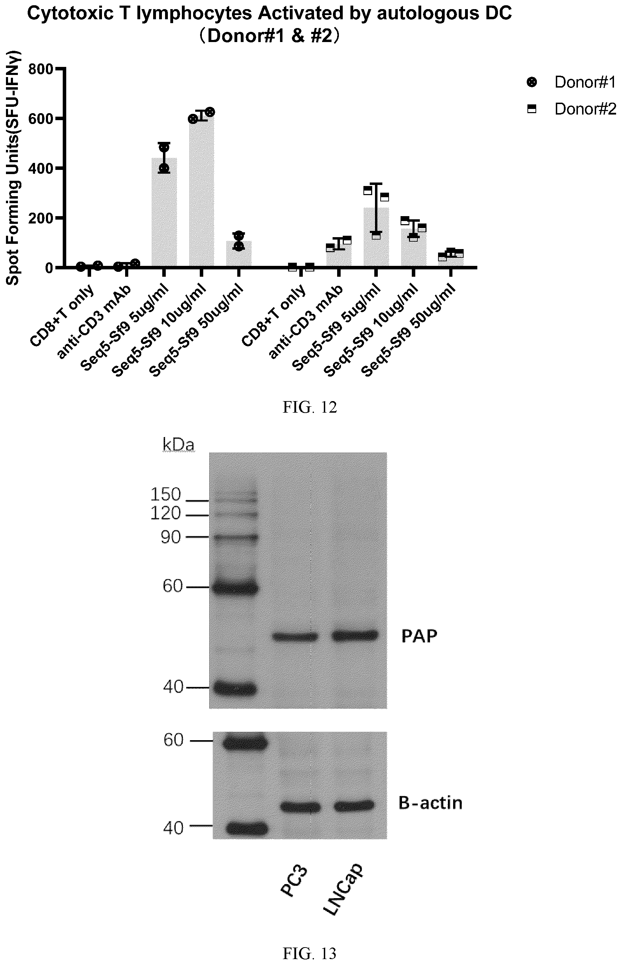

The antigen peptide/MHC-I molecule complex presented on the DC membrane surface can be directly recognized by and bind to the TCR on the surface of CD8+T cells, thereby activating the CD8+T cells to play their biological roles, in which one of the main ways is to secrete interferon γ (IFNγ). The Seq5-Sf9-loaded DCs are co-cultured with CD8+T cells from the same volunteer, and the Seq5-Sf9-loaded DCs are added again for secondary stimulation to activate a large amount of the CD8+T cells. IFNγ secreted at a single-cell level is detected by ELISPOT. The detection results are shown in FIG. 12. It can be seen that the IFNγ in the Seq5-Sf9-loaded group is significantly more than that in the volunteers' own CD8+T cells (negative control), that is, the Seq5-Sf9 can effectively promote the activation of the CD8+T cells.

### 2.5.4 Human prostate cancer cell lines expressing PAP antigen as target cells to evaluate the killing effect of CTL activated by Seq5-Sf9

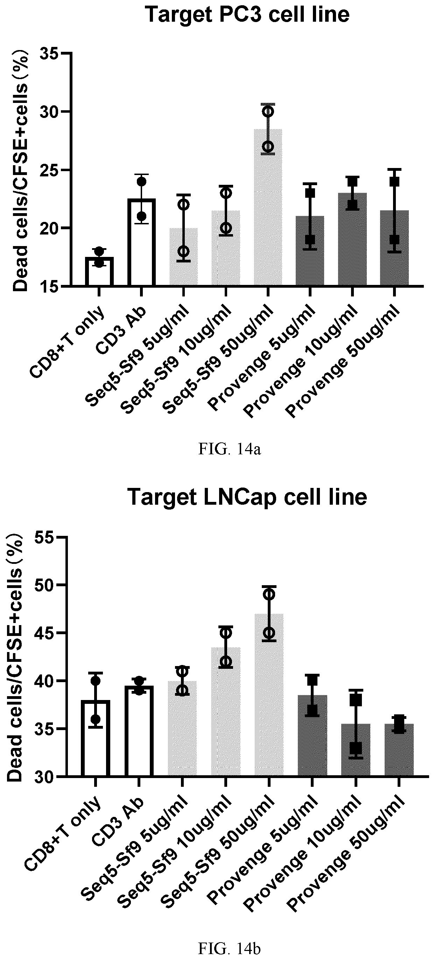

The therapeutic effect for the prostate cancer depends on whether the prostate cancer cells are sensitive to androgen. PC3 is a common androgen-independent human prostate cancer cell line, while the LNCap is androgen-dependent. Both cell lines express a certain amount of PAP, and as shown in FIG. 13, the LNCap expresses relatively more PAP protein. The above two cell lines are used as target cells and are co-cultured with effector cells (CTL activated by DCs loaded with the Seq5-Sf9). The absolute count of dead cells is calculated using CFSE and Counting Beads. As shown in FIGs. 14a-14b, the CTL activated by DCs loaded with the Seq5-Sf9 can directly kill the target cells, and the killing effect is proportional to the concentration of the Seq5-Sf9. In addition, the number of dead cells of the LNCap expressing higher amount of PAP cells is much higher than that of the PC3, verifying a targeted killing effect of the CTL activated by DCs loaded with the Seq5-Sf9, and the effect thereof being higher than that of Provenge.

### 2.6 Immunogenicity evaluation of Seq5-Sf9

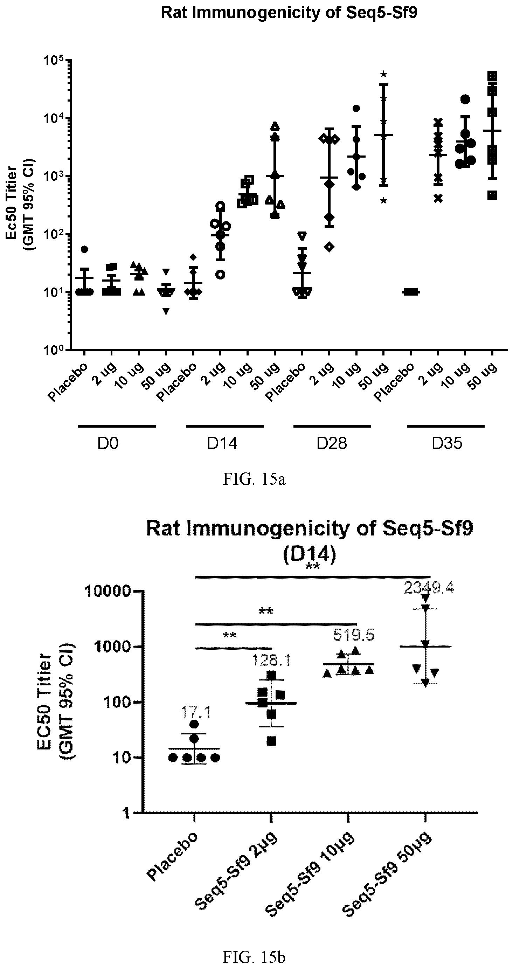

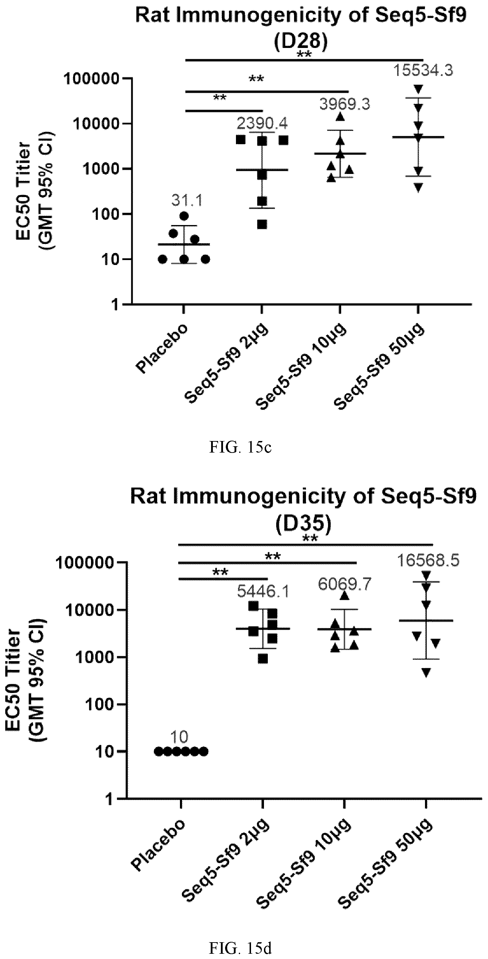

To in vivo evaluate the immune response effect of the Seq5-Sf9, 6-8 week-old Sprague Dawley (SD) male rats are immunized with the Seq5-Sf9. The SD male rats are subcutaneously injected with the Seq5-Sf9 (Placebo/rat, 2ug/rat, 10ug/rat, 50ug/rat) every two weeks, for a total of 3 injections. After immunization of the rats with the Seq5-Sf9, the rat serum is collected (on Day 14, Day 28, and Day 35 (one week after the third immunization)) for detecting Seq5-Sf9-specific antibody titer. As shown in FIGs. 15a-15d, compared with the Placebo group, the vaccine-specific serum antibodies are present at a low dose of 2ug, and the antibody titer reaches the highest level at the third injection and one week after the third injection.

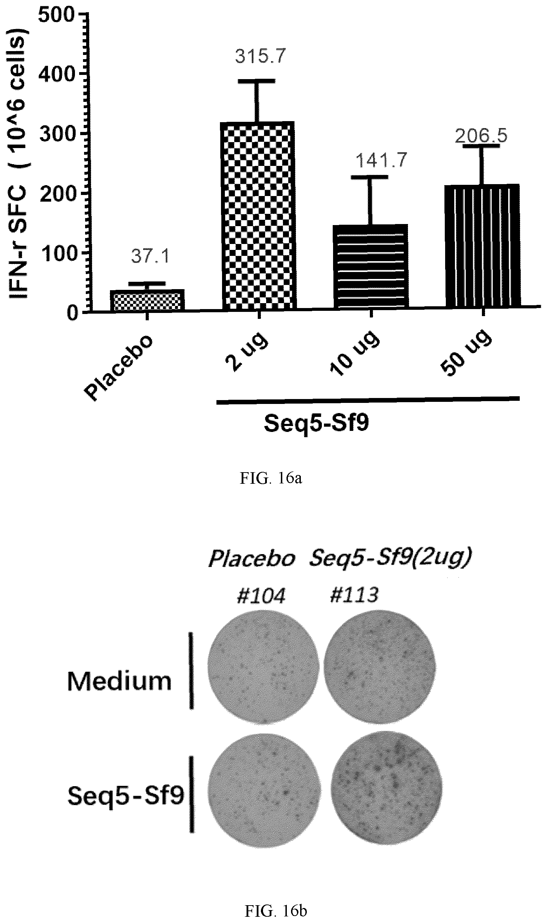

In addition, the spleen of rats on Day 35 is taken and re-stimulated with the Seq5-Sf9 in vitro. T cell activation is activated and induced, and the secretion of IFNγ by single spleen cells is detected by ELISPOT. As shown in FIGs. 16a-16b, the spleen immune cells can rapidly differentiate into cytotoxic T lymphocytes to secrete IFNγ under the secondary stimulation of the Seq5-Sf9.

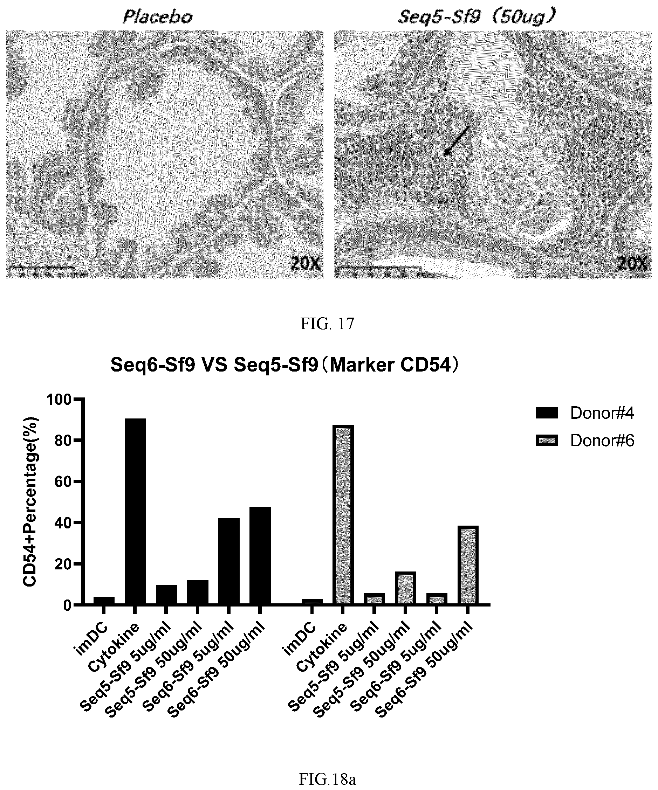

Meanwhile, the prostate tissue is observed, and the observation results are shown in FIG. 17. Inflammatory cell infiltration is also observed in the prostate tissue of the rats in the Seq5-Sf9 group.

In summary, the Seq5-Sf9 can establish a complete in-vivo immune response in the SD rats and effectively activate T cells, that is, the Seq5-Sf9 can complete in-vivo immune activation.

### 2.7 Evaluation of the immune activation effect of the fusion protein Seq6-Sf9 using DCs induced by CD14+ monocytes in human peripheral blood PBMCs

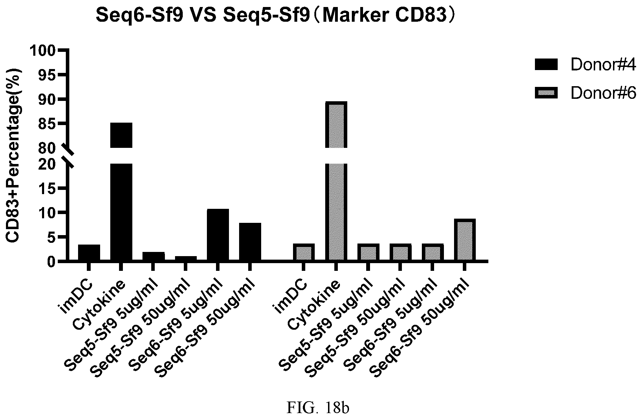

The Fc fragment of the Seq6-Sf9 is different from that of the Seq5-Sf9. To evaluate its immune activation effect, CD14+ monocytes sorted from PBMCs of two healthy human beings are induced to differentiate and are loaded with the Seq6-Sf9. Changes in surface markers of the antigen-loaded DCs are detected by the Flow cytometry. As shown in FIGs. 18a-18b, the Seq6-Sf9 increases the expression of CD54 and CD83 on the surface of DC cells and promotes DC activation. The Seq6-Sf9 has a higher expression amount of the markers than the Seq5-Sf9.

Finally, it should be noted that the above embodiments are only used to illustrate the technical solutions of this application, rather than to limit this application. Although this application has been described in detail with reference to the aforementioned embodiments, those skilled in the art should understand that the technical solutions described in the aforementioned embodiments can still be amended, or some or all of the technical features therein can be replaced equivalently. However, these amendments or replacements do not cause the essence of the corresponding technical solutions to deviate from the scope of the technical solutions of the embodiments in this application.

## Claims

1. A glycosyl-modified fusion protein, comprising a murine Fc variant and a prostate cancer tumor antigen, wherein the fusion protein enhances binding to DC and DC activation, comprising proliferation and activation of specific T cells;
the murine Fc variant is obtained by subjecting murine Fc fragment to amino acid mutation and non-mammalian glycosylation-modification;
the murine Fc variant comprises at least one of alanine at position 223, alanine at position 228, alanine at position 230, leucine at position 330, and glutamic acid at position 332;
the non-mammalian glycosylation-modification does not comprise sialic acid-modification;
a position numbering of amino acid is based on EU numbering system.

2. The fusion protein according to claim 1, wherein an amino acid sequence of the murine Fc variant is shown in SEQ ID NO: 2 or SEQ ID NO: 3.

3. The fusion protein according to claim 1 or 2, wherein in the glycosylation-modification, the glycosyl is derived from at least one of mannose, N-acetylglucosamine and fucose.

4. The fusion protein according to claim 3, wherein in the glycosylation-modification, a carbohydrate chain structure formed by linking of the glycosyl is selected from at least one of high mannose type, oligomannose type and fucose type.

5. The fusion protein according to claim 1, wherein the prostate cancer tumor antigen is PAP.

6. The fusion protein according to any one of claims 1 to 5, wherein the fusion protein further comprises a protein tag.

7. The fusion protein according to any one of claims 1 to 6, wherein the fusion protein further comprises a linking peptide.

8. A nucleic acid molecule encoding the fusion protein according to any one of claims 1 to 7.

9. A recombinant expression vector, comprising the nucleic acid molecule according to claim 8.

10. A host cell, comprising the recombinant expression vector according to claim 9.

11. A pharmaceutical composition, comprising the fusion protein according to any one of claims 1 to 7 and a pharmaceutically acceptable carrier.

12. Use of the fusion protein according to any one of claims 1 to 7 in preparation of a medicament for treating prostate cancer.

13. Use of the fusion protein according to any one of claims 1 to 7 in preparation of a medicament for treating any tumor expressing PAP tumor antigen.

14. A method for treating prostate cancer, comprising administering the fusion protein according to any one of claims 1 to 7 to a subject.
